# EUROPEAN PATENT APPLICATION

(11) **EP 4 487 767 A1**
(43) Date of publication of application: **08.01.2025**
(21) Application number: 23759967.5
(22) Date of filing: 21.02.2023
(51) Int. Cl.: A61B 5/08, A61B 5/11, G06N 20/00

(54) **ABNORMALITY DETERMINATION MODEL GENERATION METHOD, ABNORMALITY DETERMINATION DEVICE, ABNORMALITY DETERMINATION METHOD, AND TRAINED MODEL**

(30) Priority: 28.02.2022 JP 2022030136
(71) Applicant: Minebea Mitsumi Inc., Kitasaku-gun, Nagano 3890293 (JP)
(72) Inventor: FUSE Tohru, Kitasaku-gun, Nagano 389-0293 (JP); ZAITSU Yusuke, Kitasaku-gun, Nagano 389-0293 (JP)
(74) Representative: Zabel, Julia Elisabeth
(86) International application number: PCT/JP2023/006188
(87) International publication number: WO 2023/162966

(57) **Abstract**

This abnormality determination model generation method comprises: detecting the load of a subject (S) by means of load sensors (LS1, LS2, LS3, LS4) disposed in a bed (BD); creating teaching data in which a plurality of kinds of feature amounts based on the detected load are respectively associated with abnormal states; and creating a model which classifies, on the basis of the plurality of kinds of feature amounts, states of the subject into the abnormal state by supervised machine-learning using the teaching data and determining, as an explanatory variable, at least one of the plurality of kinds of feature amounts on the basis of the model; and generating an abnormal determination model which determines, by means of the machine-learning using the explanatory variable, that the subject is in the abnormal state on the basis of the explanatory variable. The plurality of kinds of feature amounts include frequency feature amounts calculated by performing a short-time Fourier transform on the load of the subject.

## Description

### Technical Field

The present invention relates to an abnormality determination model generation method, an abnormality determination device, an abnormality determination method, and a trained model.

### Background Art

There has been proposed a system for determining a state of a subject on a bed without attaching a measuring instrument to a body of the subject in medical and nursing care sites. Specifically, for example, a system is known for detecting a load of a subject on a bed via a load detector and performing determination of presence in bed or leaving bed, determination of a respiratory state, and the like based on the detected load.

PTL 1 discloses a biological information monitoring system for determining the number of subjects on a bed, a respiratory rate of a subject, and the like based on a detection value of a load detector. PTL 2 discloses a biological information monitoring system for obtaining a direction of a body axis of a subject or the like based on a detection value of a load detector.

### Citation List

### Patent Literature

PTL 1: JP 6321719 B
PTL 2: JP 6594399 B

### Summary of Invention

### Technical Problem

In the systems described in PTLs 1 and 2, it is necessary to consider an influence of a body motion of the subject on load fluctuation when performing state determination of the subject on the bed. Even when it is desired to remove an influence of load fluctuation due to body motion of the subject, it is not necessarily easy to sufficiently remove the influence.

In view of the above, an object of the present invention is to provide an abnormality determination model generation method for generating an abnormality determination model capable of suitably performing abnormality determination of a subject based on load detection.

An object of the present invention is to provide an abnormality determination device, an abnormality determination method, and a trained model capable of suitably performing abnormality determination of a subject based on load detection.

### Solution to Problem

According to a first aspect of the present invention, an abnormality determination model generation method is provided for determining a subject on a bed to be in an abnormality state, the abnormality determination model generation method including:
detecting a load of the subject by a load sensor disposed at the bed;
creating teaching data, each of a plurality of types of feature amounts based on the detected load of the subject being associated with the abnormality state in the teaching data;
creating a model for classifying a state of the subject as the abnormality state based on the plurality of types of feature amounts and determining at least one of the plurality of types of feature amounts as an explanatory variable based on the model, through supervised machine learning using the teaching data; and
generating an abnormality determination model for determining the subject to be in the abnormality state based on the explanatory variable, through machine learning using the explanatory variable, wherein
the plurality of types of feature amounts include a frequency feature amount calculated by performing short-time Fourier transform on the load of the subject.

According to a second aspect of the present invention, an abnormality determination device is provided for determining a subject on a bed to be in an abnormality state, the abnormality determination device including:
a preprocessing unit configured to calculate a frequency feature amount by performing short-time Fourier transform on a load of the subject detected by a load sensor disposed at the bed; and
an abnormality determination unit configured to store an abnormality determination model, wherein
the abnormality determination model is a trained model for determining the subject to be in the abnormality state with the frequency feature amount as an input.

According to a third aspect of the present invention, an abnormality determination method is provided for determining a subject on a bed to be in an abnormality state, the abnormality determination method including:
calculating a frequency feature amount by performing short-time Fourier transform on a load of the subject detected by a load sensor disposed at the bed; and
determining the subject to be in the abnormality state by an abnormality determination model being a trained model for determining the subject to be in the abnormality state with the frequency feature amount as an input.

According to a fourth aspect of the present invention, a trained model is provided for determining a subject on a bed to be in an abnormality state, wherein
machine learning is performed based on a frequency feature amount calculated by performing short-time Fourier transform on a load of the subject detected by a load sensor disposed at the bed, and
a computer is caused to function so as to determine the subject to be in the abnormality state based on the frequency feature amount.

According to a fifth aspect of the present invention, an abnormality determination model generation method is provided for determining a subject on a bed to be in an abnormality state, the abnormality determination model generation method comprising:
detecting a load of the subject by a load sensor disposed at the bed;
calculating a frequency feature amount by performing short-time Fourier transform on the load of the subject; and
generating an abnormality determination model for determining the subject to be in the abnormality state based on the frequency feature amount by unsupervised machine learning using the frequency feature amount, wherein
the abnormality state includes a coughing state and an apneic state, and
the frequency feature amount includes a mean amplitude value of a frequency spectrum in a first frequency band included in a band of 0.1 to 1.0 Hz and a mean amplitude value of a frequency spectrum in a second frequency band included in a band of 3.6 to 5.5 Hz.

### Advantageous Effects of Invention

According to the abnormality determination model generation method of the present invention, it is possible to generate an abnormality determination model capable of suitably performing abnormality determination of a subject based on load detection. According to the abnormality determination device, the abnormality determination method, and the trained model of the present invention, it is possible to suitably perform abnormality determination of a subject based on load detection.

### Brief Description of Drawings

FIG. 1 is a block diagram illustrating a configuration of an abnormality determination system.
FIG. 2 is an explanatory diagram illustrating an arrangement of a load sensor with respect to a bed.
FIG. 3 is a flowchart of an abnormality determination method using the abnormality determination system.
FIG. 4(a) is a graph showing an example of temporal fluctuation of a load signal. FIG. 4(b) is a graph showing an example of a frequency spectrum obtained based on the load signal of FIG. 4(a).

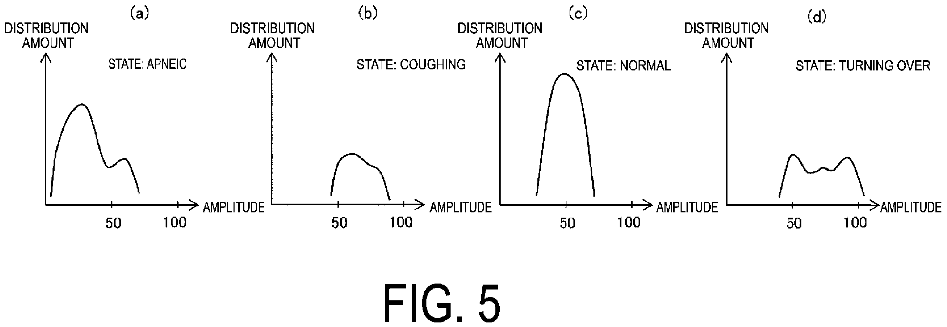

FIGS. 5(a) to 5(d) are graphs each showing the tendency of a mean value of a spectrum amplitude in a band of 0.2 Hz to 0.5 Hz of the frequency spectrum obtained based on the load signal, the horizontal axis and the vertical axis being logarithms of the spectrum amplitude. The horizontal axis represents the magnitude of the spectrum amplitude, and the vertical axis represents a distribution amount. FIG. 5(a) shows a tendency in a period of the subject being in an apneic state, FIG. 5(b) shows a tendency in a period of the subject being in a coughing state, FIG. 5(c) shows a tendency in a period of the subject being in a normal state, and FIG. 5(d) shows a tendency in a period of the subject turning over.

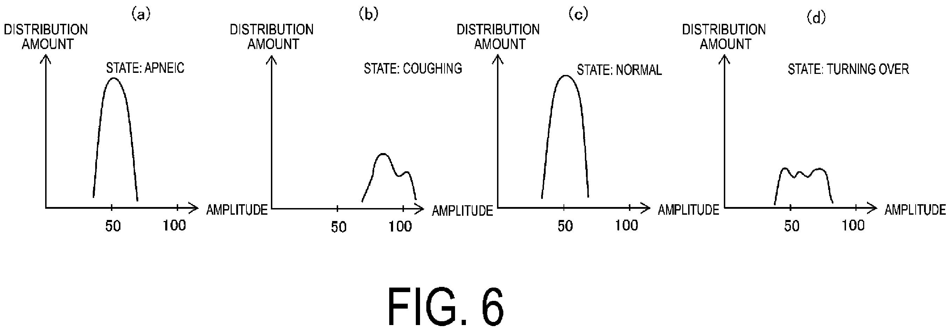

FIGS. 6(a) to 6(d) are graphs each showing the tendency of a mean value of a spectrum amplitude in a band of 4.6 Hz to 5.0 Hz of the frequency spectrum obtained based on the load signal, the horizontal axis and the vertical axis being logarithms of the spectrum amplitude. The horizontal axis represents the magnitude of the spectrum amplitude, and the vertical axis represents a distribution amount. FIG. 6(a) shows a tendency in a period of the subject being in an apneic state, FIG. 6(b) shows a tendency in a period of the subject being in a coughing state, FIG. 6(c) shows a tendency in a period of the subject being in a normal state, and FIG. 6(d) shows a tendency in a period of the subject turning over.
FIG. 7 is a schematic scatter diagram illustrating a distribution of spectrum amplitude pairs.
FIG. 8 is a flowchart of an abnormality determination model generation method.
FIG. 9 is a graph showing an example of temporal fluctuation of four load signals detected by four load sensors.
FIG. 10 is a graph showing an increase in score when coughing or apnea occurs in a subject regarding an abnormality determination model obtained by unsupervised machine learning using a random cut forest algorithm.

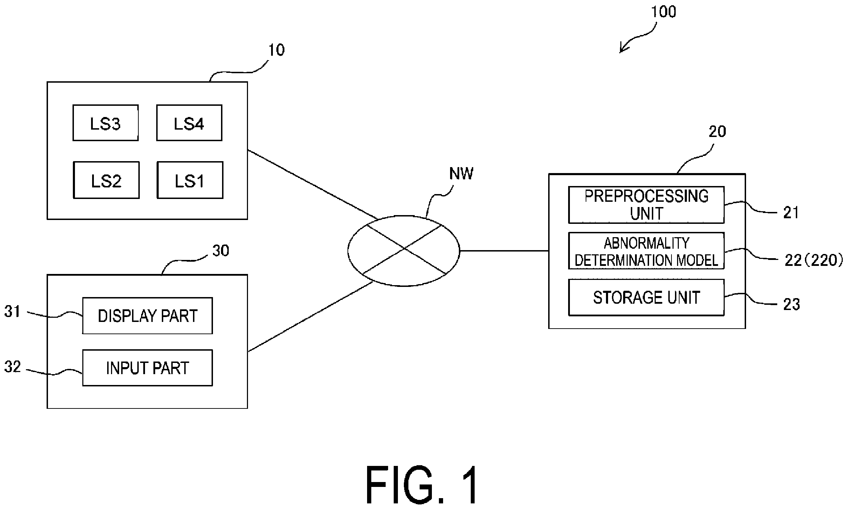

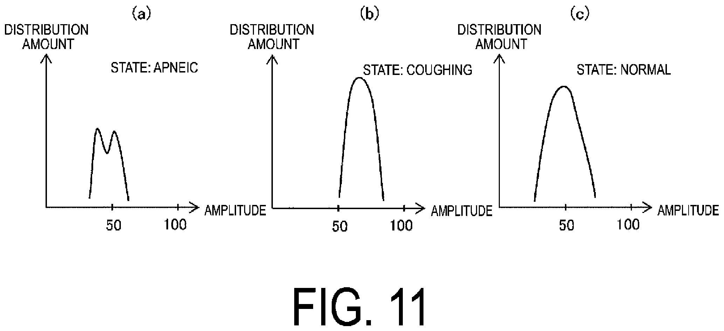

FIGS. 1 1(a) to 1 1(c) are graphs each showing the tendency of a mean value of a spectrum amplitude in a band of 3.6 Hz to 4.0 Hz of the frequency spectrum obtained based on the load signal, the horizontal axis and the vertical axis being logarithms of the spectrum amplitude. The horizontal axis represents the magnitude of the spectrum amplitude, and the vertical axis represents a distribution amount. FIG. 11(a) shows a tendency in a period of the subject being in an apneic state, FIG. 1 1(b) shows a tendency in a period of the subject being in a coughing state, and FIG. 11(c) shows a tendency in a period of the subject being in a normal state.

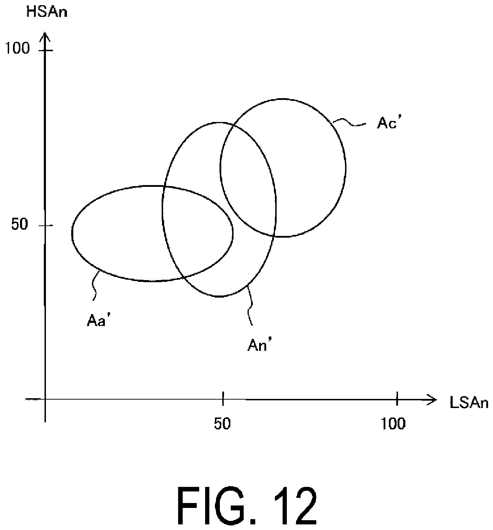

FIG. 12 is a schematic scatter diagram illustrating a distribution of spectrum amplitude pairs.

### Description of Embodiments

### Embodiments

### Abnormality Determination System and Abnormality Determination Method

An abnormality determination system 100 and an abnormality determination method according to an embodiment of the present invention will be described with reference to FIGS. 1 to 10 with an example when determination is performed by regarding a coughing state (a state of coughing) and an apneic state (a state of being apneic) as abnormality states.

As illustrated in FIG. 1, the abnormality determination system 100 mainly includes a load measurement unit 10, an abnormality determination device 20, and a terminal device 30. The load measurement unit 10, the abnormality determination device 20, and the terminal device 30 are connected to one another via a network NW such as the Internet.

The load measurement unit 10 mainly includes four load sensors LS1, LS2, LS3, and LS4. Each of the four load sensors LS1 to LS4 measures a load using a load cell having a beam shape, for example. Such a load sensor is described in, for example, J P 4829020 B and J P 4002905 B.

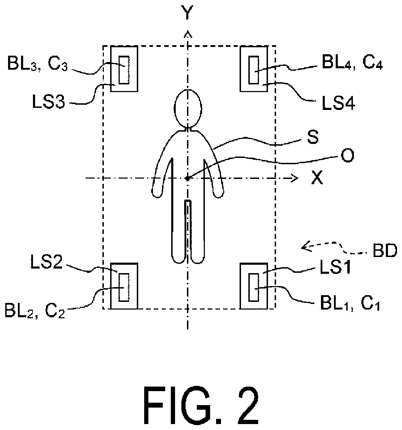

As illustrated in FIG. 2, the four load sensors LS1 to LS4 are disposed under casters C₁, C₂, C₃, and C₄ attached to lower end parts of legs BL₁, BL₂, BL₃, and BL₄, respectively, at the four corners of a bed BD used by a subject S.

The abnormality determination device 20 mainly includes a preprocessing unit 21, an abnormality determination unit 220 configured to store an abnormality determination model 22, and a storage unit 23. The abnormality determination device 20 may be configured in a single physical server or may be configured on a cloud (cloud server).

The preprocessing unit 21 calculates data (explanatory variable) to be input to the abnormality determination model 22 based on a load value received from the load measurement unit 10 via the network NW. The abnormality determination model 22 is a trained model for determining the subject S on the bed BD to be in an abnormality state based on a predetermined explanatory variable. The functions of the preprocessing unit 21 and the abnormality determination model 22 will be described later in detail. Note that in the present invention, the "model for determining the subject to be in an abnormality state" includes a model for outputting information (score) for determining the subject to be in an abnormality state.

The storage unit 23 stores various programs, various data, and the like used in the abnormality determination device 20. The storage unit 23 may also store data obtained by operation of the abnormality determination model 22. The stored data can be used for remodeling (relearning) of the abnormality determination model 22.

The terminal device 30 mainly includes a display part 31 and an input part 32.

The display part 31 displays a determination result of the abnormality determination device 20 to a user (a medical doctor, a nurse, a care worker, a caretaker at home, or the like) of the abnormality determination system 100. The display part 31 includes, for example, an image display part (a liquid crystal display or the like) and/or a voice display part (a speaker or the like).

The input part 32 is an interface for performing various inputs to the abnormality determination system 100. The input part 32 may be, for example, a press button or a touchscreen configured integrally with a liquid crystal display of the display part 31.

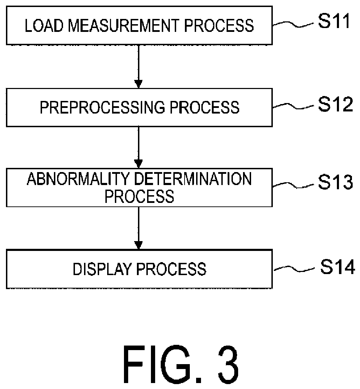

As shown in the flowchart of FIG. 3, the abnormality determination method using the abnormality determination system 100 includes a load measurement process S11, a preprocessing process S12, an abnormality determination process S13, and a display process S14.

In the load measurement process S11, the load of the subject S on the bed BD (i.e., the load applied to the bed BD by the subject S) is measured using the load sensors LS1 to LS4 of the load measurement unit 10. The load of the subject S is dispersedly applied to the load sensors LS1 to LS4 disposed under the legs BL₁ to BL₄ at the four corners of the bed BD, and is measured dispersedly by them.

Each of the load sensors LS1 to LS4 detects the load of the subject S as an analog signal. Hereinafter, the analog signals (i.e., raw data signals) detected by the load sensors LS1, LS2, LS3, and LS4 are described as analog signals s₁, s₂, s₃, and s₄, respectively.

The analog signals s₁ to s₄ are sent to the abnormality determination device 20 via a communication interface (not illustrated) of the load measurement unit 10 and the network NW.

In the preprocessing process S12, an explanatory variable to be input to the abnormality determination model 22 is calculated using the preprocessing unit 21 of the abnormality determination device 20.

The preprocessing unit 21 first receives the analog signals s₁ to s₄ sent from the load measurement unit 10 via the network NW and a communication interface (not illustrated) of the abnormality determination device 20. Then, the analog signals s₁ to s₄ are digitally converted with a sampling period of 5 milliseconds, for example, and digital signals are acquired. Hereinafter, digital signals obtained by digitally converting the analog signals s₁, s₂, s₃, and s₄ are described as load signals ss₁, ss₂, ss₃, and ss₄, respectively.

In the present embodiment, the preprocessing unit 21 calculates a low frequency band spectrum amplitude and a high frequency band spectrum amplitude based on each of the load signals ss₁ to ss₄ as explanatory variables input to the abnormality determination model 22. Hereinafter, the low frequency band spectrum amplitude based on the load signal ssₙ (n = 1, 2, 3, 4) is described as LSAn (n = 1, 2, 3, 4), and the high frequency band spectrum amplitude is described as HSAn (n = 1, 2, 3, 4).

Specifically, the low frequency band spectrum amplitude LSA1 and the high frequency band spectrum amplitude HSA1 are sequentially calculated as follows, for example.

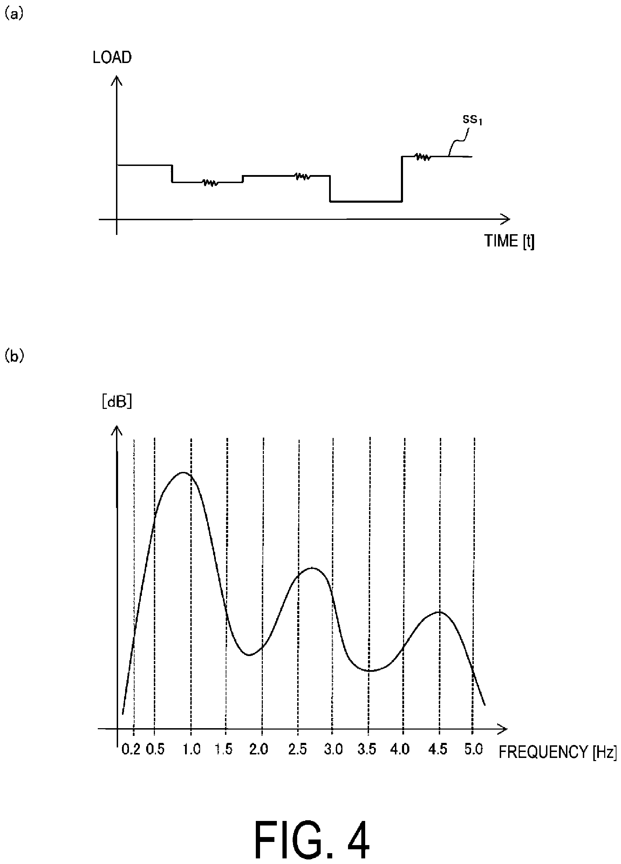

First, short-time Fourier transform is sequentially performed on the load signal ss₁ (an example is shown in FIG. 4(a)) to sequentially generate a frequency spectrum (an example is shown in FIG. 4(b)). The length of a window function of the short-time Fourier transform generally needs to be adjusted according to a targeted frequency width and a time length to be cut out. In the present embodiment, the short-time Fourier transform is executed every 10 s.

Next, for the generated frequency spectrum, a mean value of amplitudes in the frequency band of 0.2 to 0.5 Hz and a mean value of amplitudes in the frequency band of 4.6 to 5.0 Hz are calculated. The mean value of the spectrum amplitudes in the frequency band of 0.2 to 0.5 Hz thus obtained is the low frequency band spectrum amplitude LSA1, and the mean value of the spectrum amplitudes in the frequency band of 4.6 to 5.0 Hz is the high frequency band spectrum amplitude HSA1.

By performing similar calculation using the load signals ss₂ to ss₄ in place of the load signal ss₁, the low frequency band spectrum amplitudes LSA2 to LSA4 and the high frequency band spectrum amplitudes HSA2 to LSA4 are sequentially calculated.

In the abnormality determination process S13, the low frequency band spectrum amplitudes LSA1 to LSA4 and the high frequency band spectrum amplitudes HSA1 to HSA4 calculated in the preprocessing process S12 are sequentially input to the abnormality determination model 22 to sequentially execute determination as to whether or not the subject S is in an abnormality state (i.e., any one of a coughing state and an apneic state).

The abnormality determination model 22 is a trained model for outputting a score for determining the subject S to have reached a coughing state or an apneic state based on the low frequency band spectrum amplitudes LSA1 to LSA4 and the high frequency band spectrum amplitudes HSA1 to HSA4.

According to findings of the inventor of the present invention, the low frequency band spectrum amplitude LSAn tends to show a relatively small value when the subject S is in an apneic state. For example, in FIGS. 5(a) to 5(d), LSAn when the subject S is in an apneic state is distributed in large numbers in regions of 20 to 40. This distribution range is on a lower amplitude side (small value side) than the distribution range of LSAn when the subject S is in a coughing state (FIG. 5(b)), when the subject S is in a normal state (FIG. 5(c)), and when the subject S is turning over (FIG. 5(d)). Note that here, the "normal state" means apnea, coughing, and turning over are not occurring in the subject S.

Similarly, according to findings of the inventor of the present invention, the high frequency band spectrum amplitude HSAn tends to show a relatively large value when the subject S is in a coughing state. For example, in FIGS. 6(a) to 6(d), HSAn when the subject S is in a coughing state is distributed in large numbers in regions of 60 to 100. This distribution range is on a higher amplitude side (large value side) than the distribution range of HSAn when the subject S is in an apneic state (FIG. 6(a)), when the subject S is in a normal state (FIG. 6(c)), and when the subject S is turning over (FIG. 6(d)).

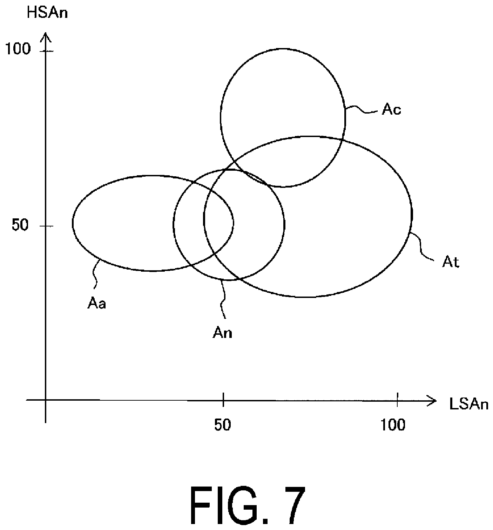

Therefore, when a scatter diagram of a spectrum amplitude pair PSAn (i.e., a pair of the low frequency band spectrum amplitude LSAn and the high frequency band spectrum amplitude HSAn based on a load signal detected at the same time) is created with the horizontal axis representing the magnitude of the low frequency band spectrum amplitude LSAn and the vertical axis representing the magnitude of the high frequency band spectrum amplitude HSAn, a region Aa is positioned in a region shifted to a small value side of the low frequency band spectrum amplitude LSAn with respect to a region An and a region At (FIG. 7). In the region Aa, the spectrum amplitude pair PSAn when the subject S is in an apneic state is distributed. In the region An, the spectrum amplitude pair PSAn when the subject S is in a normal state is distributed. In the region At, the spectrum amplitude pair PSAn when the subject S is turning over is distributed.

A region Ac is positioned in a region shifted to a large value side of the high frequency band spectrum amplitude HSAn with respect to the region An and the region At (FIG. 7). In the region Ac, the spectrum amplitude pair PSAn when the subject S is in a coughing state is distributed. In the region An, the spectrum amplitude pair PSAn when the subject S is in a normal state is distributed. In the region At, the spectrum amplitude pair PSAn when the subject S is turning over is distributed.

Based on this tendency, by machine learning with the low frequency band spectrum amplitudes LSA1 to LSA4 and the high frequency band spectrum amplitudes HSA1 to HSA4 as learning data, the abnormality determination model 22, being a trained model for determining the subject S to be in an abnormality state, can be generated. Specifically, the trained model outputs a high score when the subject S is in a coughing state or an apneic state. Therefore, for example, the abnormality determination unit 220 can determine the subject S to be in an abnormality state (i.e., any one of a coughing state and an apneic state) by comparing the score with a predetermined value (the generation method of the abnormality determination model 22 will be described later in detail).

In the display process S14, a determination result of the abnormality determination model 22 (abnormality determination unit 220) is displayed on the display part 31 of the terminal device 30. The display can be made in any aspect. For example, when the abnormality determination model 22 determines the subject S to have reached an abnormality state, the display part 31 displays that effect by an image and/or voice. Note that the display process S14 may be omitted, and the determination result may be only stored in the storage unit 23.

The abnormality determination system 100 and the abnormality determination method of the present embodiment perform abnormality determination of the subject S using the abnormality determination model 22 (details of the generation method will be described later) generated in consideration also of an influence of a load change due to body motion. Therefore, the abnormality determination of the subject S can be suitably performed.

More specifically, the abnormality determination system 100 and the abnormality determination method of the present embodiment create an abnormality determination model based on a tendency appearing in a frequency spectrum regardless of the presence or absence of body motion of the subject when the subject coughs or when the subject becomes apneic, and determine the subject to have reached a coughing state or an apneic state based on the abnormality determination model. Therefore, even in a period of body motion occurring in the subject S and the load signals ss₁ to ss₄ fluctuating due to a factor not related to respiration of the subject, it is possible to determine whether or not the subject S has reached a coughing state or an apneic state based on load measurement. Note that here, the "body motion" includes a large body motion (e.g., turning over) accompanied by movement of a torso part and a small body motion accompanied by movement of a head part and/or four limbs.

In the above embodiment, the abnormality state includes both the coughing state and the apneic state, and the abnormality determination system 100 determines the subject S to be in the abnormality state when the subject S has reached any of the coughing state and the apneic state, but the present invention is not limited to this. The abnormality determination system 100 may be configured not to determine the subject S to be abnormal when in the apneic state but to determine the subject S to be abnormal when in the coughing state, or may be configured not to determine the subject S to be abnormal when in the coughing state but to determine the subject S to be abnormal when in the apneic state.

The state regarded as abnormal by the abnormality determination system 100 can be newly added by associating the analog signals s₁, s₂, s₃, and s₄ (or the load signals ss₁, ss₂, ss₃, and ss₄), being detection values of the load sensors LS1 to LS4, with the state of the subject capable of being classified on a time axis, and performing labeling as described later.

### Abnormality Determination Model Generation Method

Next, the abnormality determination model generation method according to an embodiment of the present invention will be described.

As the abnormality determination model 22 included in the abnormality determination device 20 of the abnormality determination system 100, different models are generated according to the content of the state regarded as abnormal by the abnormality determination system 100. Hereinafter, an abnormality determination model generation method according to the content of a state regarded as abnormal will be described.

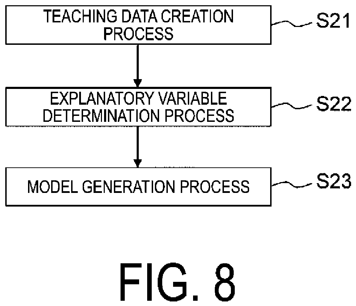

As shown in the flowchart of FIG. 8, the abnormality determination model generation method of the present embodiment includes a teaching data creation process S21, an explanatory variable determination process S22, and a model generation process S23.

In the teaching data creation process S21, teaching data used in the explanatory variable determination process S22 is created by the following procedure as an example.

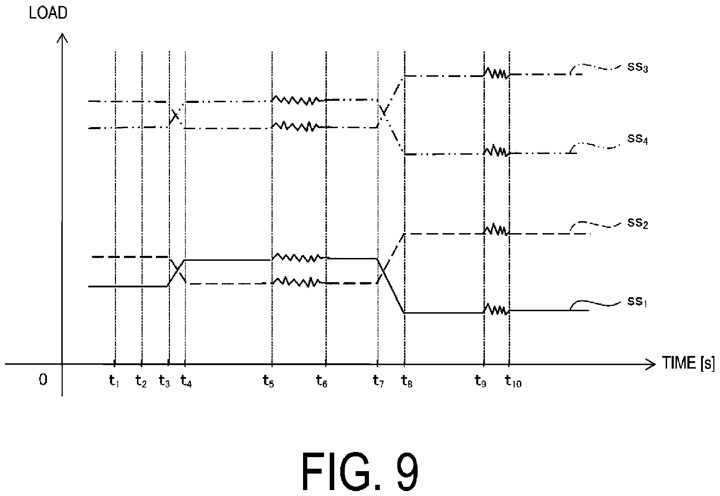

First, in a state of the subject S existing on the bed BD, measurement of the load of the subject S by the load sensors LS 1 to LS4 and photographing of the subject S on the bed BD are performed in parallel over a predetermined period, and the load signals ss₁ to ss₄ and an image (moving image) of the subject S are stored. An example of the stored load signals ss₁ to ss₄ is as shown in FIG. 9. At this time, voice of the subject S may be recorded.

Next, the stored load signals ss₁ to ss₄ are labeled (tagged) based on the stored image of the subject S. The labeling is performed by associating the type of the state (motion) of the subject S and start time and end time of the state with the load signals ss₁ to ss₄.

The types of states to be labeled are, by way of example, "apneic", "coughing", "normal", and "turning over". In the example shown in FIG. 9, a period from time t₁ to time t₂ is labeled as "apneic", a period from time t₃ to time t₄ and a period from time t₇ to time t₈ as "turning over", a period from time t₅ to time t₆ and a period from time t₉ to time t₁₀ as "coughing", and other periods as "normal".

In place of or in addition to "apneic", "coughing", "normal", and "turning over", the types of states to be labeled may include at least one of "getting into bed" (movement of the subject from under the bed to on the bed), "leaving bed" (movement of the subject from on the bed to under the bed), "edge sitting position" (state of the subject sitting on an edge of the bed), "sitting position" (state of the subject sitting on the bed), "body motion other than turning over", "angle change of a reclining bed" (ascending or descending), dangerous motion on the bed (such as jumping out from the bed fence), "meal", "supine", "right side lying", "left side lying", and "prone".

Next, a plurality of feature amounts are created based on the stored load signals ss₁ to ss₄. The feature amounts used in the present embodiment can include the following feature amounts.

### (1) Load Signals ss₁ to ss₄

The load signals ss₁ to ss₄ are directly used as the feature amounts.

### (2) Spectrum amplitude SAn_{X-Y} in each frequency band

Short-time Fourier transform is sequentially performed on the load signal ssₙ to sequentially generate a frequency spectrum (an example is shown in FIG. 4(b)). The length of the window function of the short-time Fourier transform can be, for example, about 10 s. The short-time Fourier transform may be executed, by way of example, every 5 s to 15 s. The window functions of time-consecutive two short-time Fourier transforms may partially overlap each other. The length of the window function of the short-time Fourier transform is known to be correlated with the time resolution and the frequency resolution. That is, if the time length of the window function is lengthened, the time resolution becomes low and the frequency resolution becomes high, and if the time length of the window function is shortened, the time resolution becomes high and the frequency resolution becomes low. As a technique for improving such characteristics, suppression of side lobe by introducing the window function is known. Generalized harmonic analysis (GHA) and short-time Fourier transform using a plurality of window lengths (multi-windows STFT) are known. Using these techniques, the frequency spectrum may be analyzed and the explanatory variables may be determined.

For each of the generated frequency spectra, the frequency is divided into 10 bands, and a mean value of the spectrum amplitudes in each band is calculated. In the present embodiment, as an example, the mean value of the spectrum amplitudes in each of the bands of 0.2 to 0.5 Hz, 0.6 to 1.0 Hz, 1.1 to 1.5 Hz, 1.6 to 2.0 Hz, 2.1 to 2.5 Hz, 2.6 to 3.0 Hz, 3.1 to 3.5 Hz, 3.6 to 4.0 Hz, 4.1 to 4.5 Hz, and 4.6 to 5.0 Hz is calculated.

Hereinafter, the mean value of the spectrum amplitudes in the frequency band of X to Y Hz of the frequency spectrum generated based on the load signal ssₙ (n = 1, 2, 3, 4) is described as a spectrum amplitude SAn_{X-Y} (n = 1, 2, 3, 4). A spectrum amplitude SAn_{0.2-0.5} is a mean value of the spectrum amplitudes in the frequency band of 0.2 to 0.5 Hz and is equal to the above-described low frequency band spectrum amplitude LSAn. A spectrum amplitude SAn_{4.6-5.0} is a mean value of the spectrum amplitudes in the frequency band of 4.6 to 5.0 Hz and is equal to the high frequency band spectrum amplitude HSAn.

### (3) Position of center of gravity

A position of center of gravity of the subject S on the bed BD is calculated based on the load signals ss₁ to ss₄. Any known method can be used for calculation of the position of center of gravity. An example of a calculation method of the position of center of gravity is described in J P 6321719 B (PTL 1).

### (4) Respiratory waveform

A center of gravity G of the subject S vibrates in a direction along the body axis (spine) of the subject S in response to respiration of the subject S (hereinafter, the vibration is called "respiratory vibration"). The respiratory waveform indicates a respiratory vibration, and the horizontal axis represents time and the vertical axis represents amplitude of the respiratory vibration. An example of a drawing method of a respiratory waveform is described in J P 6661173 B.

### (5) Respiratory rate

The respiratory rate of the subject S is calculated based on, for example, a respiratory waveform. Since one cycle of the respiratory waveform corresponds to respiration (expiration and inspiration) of one time, a respiratory rate B for 1 minute is estimated as B = 60/T, a peak to peak distance of the respiratory waveform being T.

### (6) Respiratory amplitude value

The respiratory rate of the subject S is calculated based on, for example, a respiratory waveform. The amplitude of the respiratory waveform can be estimated as a respiratory amplitude value.

### (7) Heart rate waveform

As an example, a waveform obtained by separating components included in the frequency band (about 0.5 Hz to about 3.3 Hz) of the heart rate from each of the load signals ss₁ to ss₄ can be a heart rate waveform.

### (8) Activity index (ACI)

The ACI is an index indicating the activity of the subject. For example, the ACI is calculated as a time integrated value in a predetermined period (e.g., 20 seconds) of a simple mean value of standard deviations σ₁ to σ₄, standard deviations of the temporal fluctuation of the load signals ss₁, ss₂, sss, and ss₄ being σ₁, σ₂, σ₃, and σ₄, respectively. In place of the standard deviations σ₁ to σ₄, values obtained by dividing the standard deviations σ₁ to σ₄ by the amplitude of the respiratory waveform may be used. Further details of the ACI are described in J P 6661173 B.

### (9) Total load

It can be calculated as a sum of the load values indicated by the load signals ss₁ to ss₄.

### (10) Others

In addition, it is possible to create and use a predetermined feature amount estimated to be related to a state regarded as abnormal according to the state.

As described above, a data set is created as teaching data. In the data set, a combination (hereinafter, called a "feature amount set FS") of the plurality of feature amounts obtained based on the load signals ss₁ to ss₄ is the input and the state of the subject S labeled to the load signals ss₁ to ss₄ is the output. Since each feature amount is created based on the load signals ss₁ to ss₄, each feature amount is associated with the state of the subject S labeled to the load signals ss₁ to SS4.

In the explanatory variable determination process S22, at least one feature amount is selected from the feature amount set FS included in the teaching data created in the teaching data creation process S21, and is determined as an explanatory variable to be used in the model generation process S23 described later.

As an example, the selection of the feature amount (determination of the explanatory variable) can be performed using a gradient boosting technique (specifically, for example, XGBoost, LightGBM, CatBoost, and the like) based on a decision tree algorithm. Note that logistic regression may be used in place of the gradient boosting technique.

Specifically, training data having the feature amount set FS as an input and a predetermined state of the subject S as an output is created using the teaching data created in the teaching data creation process S21. The predetermined state includes a state to be determined as an abnormality state by the generated model, and at least one of a plurality of labeled states is selected. Then, using the training data, a trained model for classifying the state of the subject S into the predetermined state using the feature amount set FS as an input is generated by the gradient boosting technique. Thereafter, a contribution degree of each feature amount in the generated trained model is calculated based on an importance degree of a decision tree algorithm, and one or a plurality of feature amounts having a high contribution degree are determined as explanatory variables to be used in the model generation process S23.

In the model generation process S23, training data with the one or the plurality of feature amounts selected in the explanatory variable determination process S22 as explanatory variables is created, and the abnormality determination model 22 is generated by unsupervised learning using the training data.

The training data is newly created from the separately measured load signals ss₁ to ss₄. When an imbalance such as a small feature amount corresponding to a certain state occurs, oversampling may be performed by a known technique such as SMOTE. As the unsupervised machine learning algorithm, in addition to random cut forest, random forest, clustering, principal component analysis, association analysis, generative adversarial network, Gaussian mixture model (GMM), local outlier factor (LOF), isolation forest, kernel density estimation, and the like can be used.

### Examples

The abnormality determination model was generated by the above-described abnormality determination model generation method. In the abnormality determination model, the coughing state and the apneic state are regarded as abnormality states.

In the teaching data creation process S21, teaching data was created. In the teaching data, the feature amount set FS including 170 types of feature amounts including (1) the load signals ss₁ to ss₄, (2) the spectrum amplitude SAn_{X-Y} of each frequency band, (3) the position of center of gravity, (4) the respiratory waveform, (5) the respiratory rate, (7) the heart rate waveform, (8) the ACI, and (9) the total load described above are inputs, and "coughing state", "apneic state", "normal state", and "turning over" are outputs.

In the explanatory variable determination process S22, a trained model for classifying the state of the subject S into a "coughing state", an "apneic state", a "normal state", and "turning over" using the feature amount set FS as an input was generated by the gradient boosting technique. Then, when the contribution degree of each feature amount in the generated trained model was calculated, it was confirmed that the contribution degree of the spectrum amplitude SAn_{0.2-0.5} (n = 1, 2, 3, 4) and the spectrum amplitude SAn_{4.6-5.0} (n = 1, 2, 3, 4) was high. Therefore, the spectrum amplitude SAn_{0.2-0.5} (n = 1, 2, 3, 4) and the spectrum amplitude SAn_{4.6-5.0} (n = 1, 2, 3, 4) were determined as explanatory variables.

In the model generation process S23, the data set including the spectrum amplitude SAn_{0.2-0.5} (n = 1, 2, 3, 4) and the spectrum amplitude SAn_{4.6-5.0} (n = 1, 2, 3, 4) as explanatory variables was used as training data, and an abnormality determination model was generated by a random cut forest algorithm.

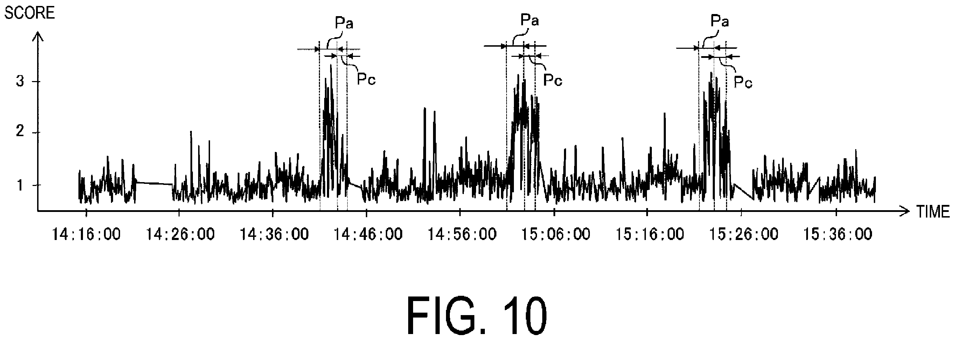

FIG. 10 shows an example of the score output when the spectrum amplitude SAn_{0.2-0.5} (n = 1, 2, 3, 4) and the spectrum amplitude SAn_{4.6-5.0} (n = 1, 2, 3, 4) were input to the generated trained model. The score output from the trained model increases in a period Pc of the subject S being in a coughing state and a period Pa of the subject S being in an apneic state. Therefore, for example, it is possible to determine the subject S to be in an abnormality state (i.e., a coughing state or an apneic state) by comparing the score with a predetermined threshold.

In the abnormality determination model generation method of the present embodiment, a feature amount having a large contribution degree in classification into a state regarded as abnormal in a decision tree model is selected from among a large number of feature amounts based on load detection, and the abnormality determination model is generated using this as an explanatory variable. Therefore, the abnormality determination model generation method of the present embodiment can generate an abnormality determination model capable of suitably performing abnormality determination of a subject based on load detection.

More specifically, in the abnormality determination model generation method of the present embodiment, a feature amount having a large contribution degree in classification into the state regarded as abnormal in the decision tree model is selected from among a large number of feature amounts including the influence of the load change due to body motion, and the abnormality determination model is generated with this as an explanatory variable. Therefore, it is possible to generate the abnormality determination model capable of performing the abnormality determination even during the period of the body motion occurring in the subject.

In the abnormality determination model generation method of the present embodiment, the spectrum amplitude SAn_{X-Y} is included in the feature amount set FS of the teaching data. In the spectrum amplitude SAn_{X-Y}, since the feature corresponding to each state of the subject S is easily maintained regardless of the presence or absence of body motion, a more appropriate feature amount can be determined as an explanatory variable in the explanatory variable determination process S22.

Note that in a case of generating the abnormality determination model 22 not regarding both the coughing state and the apneic state as abnormality states and only regarding the coughing state as an abnormality state, for example, a supervised machine learning algorithm is used in the model generation process S23. As the teaching data, for example, from the teaching data created in the teaching data creation process S21, teaching data can be created and used. In the teaching data, the explanatory variable is the spectrum amplitude SAn_{0.2-0.5} (n = 1, 2, 3, 4) and the spectrum amplitude SAn_{4.6-5.0} (n = 1, 2, 3, 4) and an objective variable is the coughing state. As the supervised machine learning algorithm, a naive Bayes classifier, a decision tree, a support vector machine (SVM), a neural network, k-nearest neighbors algorithm, association analysis, gradient boosting, logistics regression, and the like can be used.

In a case of generating the abnormality determination model 22 not regarding both the coughing state and the apneic state as abnormality states and only regarding the apneic state as an abnormality state, for example, a supervised machine learning algorithm is used in the model generation process S23. As the teaching data, for example, from the teaching data created in the teaching data creation process S21, teaching data can be created and used. In the teaching data, the explanatory variable is the spectrum amplitude SAn_{0.2-0.5} (n = 1, 2, 3, 4) and the spectrum amplitude SAn_{4.6-5.0} (n = 1, 2, 3, 4) and an objective variable is the apneic state. As the supervised machine learning algorithm, a naive Bayes classifier, a decision tree, a support vector machine (SVM), a neural network, k-nearest neighbors algorithm, association analysis, gradient boosting, logistics regression, and the like can be used.

### Modifications

In the embodiment described above, the following modified aspects can also be used.

In the abnormality determination system 100 of the above embodiment, the mean value of the spectrum amplitudes in the frequency band of 0.2 to 0.5 Hz is the low frequency band spectrum amplitude LSAn, and the mean value of the spectrum amplitudes in the frequency band of 4.6 to 5.0 Hz is the high frequency band spectrum amplitude HSAn, but the present invention is not limited to this. The low frequency band spectrum amplitude LSAn may be a mean value of spectrum amplitudes of any bands included in the band of 0.1 to 1.0 Hz. Similarly, the high frequency band spectrum amplitude HSAn may be a mean value of spectrum amplitudes of any bands included in the 3.6 to 5.5 Hz band, the 4.0 to 5.5 Hz band, and the like.

As one modification, a case of the spectrum amplitude SAn_{3.6-4.0} in the frequency band of 3.6 to 4.0 Hz being used as the high frequency band spectrum amplitude HSAn will be described.

In FIGS. 11(a) to 11(c), HSAn when the subject S is in an apneic state is distributed in large numbers in regions of 40 to 60, HSAn when the subject S is in a coughing state is distributed in large numbers in regions of 50 to 80, and HSAn when the subject S is in a normal state is distributed in large numbers in regions of 30 to 70.

Therefore, when a scatter diagram (FIG. 12) of the spectrum amplitude pair PSAn (i.e., a pair of the low frequency band spectrum amplitude LSAn and the high frequency band spectrum amplitude HSAn based on a load signal detected at the same time) is created with the horizontal axis representing the magnitude of the low frequency band spectrum amplitude LSAn (equal to the spectrum amplitude SAn_{0.2-0.5}) and the vertical axis representing the magnitude of the high frequency band spectrum amplitude HSAn (equal to the spectrum amplitude SAn_{3.6-4.0}), a region Aa' is positioned in a region shifted to a small value side of the low frequency band spectrum amplitude LSAn with respect to a region An'. In the region Aa', the spectrum amplitude pair PSAn when the subject S is in an apneic state is distributed. In the region An', the spectrum amplitude pair PSAn when the subject S is in a normal state is distributed. A region Ac' is positioned in a region shifted to a large value side of the low frequency band spectrum amplitude LSAn with respect to the region An'. In the region Ac', the spectrum amplitude pair PSAn when the subject S is in a coughing state is distributed. In the region An', the spectrum amplitude pair PSAn when the subject S is in a normal state is distributed.

Based on this tendency, by machine learning using the spectrum amplitude SAn_{0.2-0.5} as the low frequency band spectrum amplitude LSAn and the spectrum amplitude SAn_{3.6-4.0} as the high frequency band spectrum amplitude HSAn, the abnormality determination model 22 for determining the subject S to be in an abnormality state can be generated.

Alternatively, the spectrum amplitude SAn_{3.6-4.0} in the frequency band of 3.6 to 4.0 Hz may be used as a middle frequency band spectrum amplitude MSAn. In this case, the abnormality determination model 22 may be generated with the low frequency band spectrum amplitude LSAn (n = 1, 2, 3, 4), the middle frequency band spectrum amplitude MSAn (n = 1, 2, 3, 4), and the high frequency band spectrum amplitude HSAn (n = 1, 2, 3, 4) as explanatory variables. This can further improve the determination accuracy of the abnormality determination model 22. Note that the middle frequency band spectrum amplitude MSAn can be a mean value of spectrum amplitudes in any frequency band higher than the low frequency band spectrum amplitude LSAn. The high frequency band spectrum amplitude HSAn can be a mean value of spectrum amplitudes in any frequency band higher than the middle frequency band spectrum amplitude MSAn.

The abnormality determination system 100 of the above embodiment may be configured to use, as explanatory variables, at least one of the low frequency band spectrum amplitudes LSA1 to LSA4 and at least one of the high frequency band spectrum amplitudes HSA1 to HSA4.

In the abnormality determination model generation method of the above embodiment, the range of the target frequency band can be appropriately changed when the spectrum amplitude SAn_{X-Y} is calculated. The target frequency band may be, for example, in a range of 0.2 Hz to 4 Hz or may be in a range of 0.2 Hz to 6 Hz. The frequency band division is not limited to 10 division and may be discretionary.

In the abnormality determination model generation method of the above embodiment, a feature amount different from the spectrum amplitude SAn_{X-Y} may be calculated by performing short-time Fourier transform on the load signal ssₙ. Specifically, for example, a mean value of a multi vector auto-regressive model of a result of performing the short-time Fourier transform on the load signal ssₙ may be a feature amount.

In the abnormality determination model generation method of the above embodiment, when the explanatory variable is known, the explanatory variable determination process S22 can be omitted.

In the abnormality determination system 100 of the above embodiment, the load measurement unit 10 may measure the load of the subject S by a plurality of pressure-sensitive sensors (pressure sensors) arranged in a matrix under a sheet in place of the load sensors LS 1 to LS4.

The abnormality determination system 100 of the above embodiment does not necessarily include all of the load sensors LS1 to LS4, and may include only any one of them. The load detectors do not necessarily need to be positioned at the four corners of the bed, and can be disposed at any position so that the load of the subject on the bed and the fluctuation of the load can be detected. The load sensors LS1 to LS4 are not limited to load sensors using load cells having a beam shape, and for example, force sensors can also be used.

The abnormality determination system 100 of the above embodiment needs not include the terminal device 30. The abnormality determination device 20 is not limited to the aspect of being connected to the load measurement unit 10 via the network, and may be disposed in the vicinity of the load measurement unit 10 and the bed BD (e.g., in the same room) and connected to the load measurement unit 10 by wiring.

The abnormality determination model 22 of the above embodiment may be recorded on a non-transitory computer-readable recording medium. By reading the recording medium with a computer, the computer can be caused to execute the abnormality determination process S13. That is, the trained model such as the abnormality determination model 22 can be recorded on the recording medium as a program.

As long as the features of the present invention are maintained, the present invention is not limited to the embodiment described above, and other forms considered within the scope of the technical concept of the present invention are also included within the scope of the present invention.

### Reference Signs List

10 Load measurement unit;
20 Abnormality determination device;
21 Preprocessing unit;
22 Abnormality determination model;
220 Abnormality determination unit;
30 Terminal device;
BD Bed;
LS1 to LS4 Load sensors;
S Subject

## Claims

1. An abnormality determination model generation method for determining a subject on a bed to be in an abnormality state, the abnormality determination model generation method comprising:
detecting a load of the subject by a load sensor disposed at the bed;
creating teaching data, each of a plurality of types of feature amounts based on the detected load of the subject being associated with the abnormality state in the teaching data;
creating a model for classifying a state of the subject as the abnormality state based on the plurality of types of feature amounts and determining at least one of the plurality of types of feature amounts as an explanatory variable based on the model, through supervised machine learning using the teaching data; and
generating an abnormality determination model for determining the subject to be in the abnormality state based on the explanatory variable, through machine learning using the explanatory variable, wherein
the plurality of types of feature amounts include a frequency feature amount calculated by performing short-time Fourier transform on the load of the subject.

2. The abnormality determination model generation method according to claim 1, wherein the machine learning using the explanatory variable is unsupervised machine learning.

3. The abnormality determination model generation method according to claim 1 or 2, wherein the frequency feature amount is a mean amplitude value in a predetermined frequency band of a frequency spectrum.

4. The abnormality determination model generation method according to claim 3, wherein the mean amplitude value in the predetermined frequency band includes a mean amplitude value in a first frequency band and a mean amplitude value in a second frequency band at a higher frequency side than the first frequency band.

5. The abnormality determination model generation method according to claim 4, wherein the first frequency band is included in a band of 0.1 to 1.0 Hz and the second frequency band is included in a band of 3.6 to 5.5 Hz.

6. The abnormality determination model generation method according to claim 4 or 5, wherein the mean amplitude value in the predetermined frequency band further includes a mean amplitude value in a third frequency band at a higher frequency side than the first frequency band and at a lower frequency side than the second frequency band.

7. The abnormality determination model generation method according to any one of claims 1 to 6, wherein the explanatory variable includes the frequency feature amount.

8. The abnormality determination model generation method according to any one of claims 1 to 7, wherein the abnormality state includes a coughing state and/or an apneic state.

9. An abnormality determination device for determining a subject on a bed to be in an abnormality state, the abnormality determination device comprising:
a preprocessing unit configured to calculate a frequency feature amount by performing short-time Fourier transform on a load of the subject detected by a load sensor disposed at the bed; and
an abnormality determination unit configured to store an abnormality determination model, wherein
the abnormality determination model is a trained model for determining the subject to be in the abnormality state with the frequency feature amount as an input.

10. The abnormality determination device according to claim 9, wherein the frequency feature amount is a mean amplitude value in a predetermined frequency band of a frequency spectrum.

11. The abnormality determination device according to claim 10, wherein the mean amplitude value in the predetermined frequency band includes a mean amplitude value in a first frequency band and a mean amplitude value in a second frequency band at a higher frequency side than the first frequency band.

12. The abnormality determination device according to claim 11, wherein the first frequency band is included in a band of 0.1 to 1.0 Hz, and a second frequency band is included in a band of 3.6 to 5.5 Hz.

13. The abnormality determination device according to claim 11 or 12, wherein the mean amplitude value in the predetermined frequency band further includes a mean amplitude value in a third frequency band at a higher frequency side than the first frequency band and at a lower frequency side than the second frequency band.

14. The abnormality determination device according to any one of claims 9 to 13, wherein the abnormality state includes a coughing state and/or an apneic state.

15. An abnormality determination system comprising:
a load sensor disposed at a bed;
the abnormality determination device according to any one of claims 9 to 14; and
a display part configured to perform predetermined display based on a determination result of the abnormality determination device.

16. An abnormality determination method for determining a subject on a bed to be in an abnormality state, the abnormality determination method comprising:
calculating a frequency feature amount by performing short-time Fourier transform on a load of the subject detected by a load sensor disposed at the bed; and
determining the subject to be in the abnormality state through an abnormality determination model being a trained model for determining the subject to be in the abnormality state with the frequency feature amount as an input.

17. The abnormality determination method according to claim 16, wherein the frequency feature amount is a mean amplitude value in a predetermined frequency band of a frequency spectrum.

18. The abnormality determination method according to claim 17, wherein the mean amplitude value in the predetermined frequency band includes a mean amplitude value in a first frequency band and a mean amplitude value in a second frequency band at a higher frequency side than the first frequency band.

19. The abnormality determination method according to claim 18, wherein the first frequency band is included in a band of 0.1 to 1.0 Hz and the second frequency band is included in a band of 3.6 to 5.5 Hz.

20. The abnormality determination method according to claim 18 or 19, wherein the mean amplitude value in the predetermined frequency band further includes a mean amplitude value in a third frequency band at a higher frequency side than the first frequency band and at a lower frequency side than the second frequency band.

21. The abnormality determination method according to any one of claims 16 to 20, wherein the abnormality state includes a coughing state and/or an apneic state.

22. A trained model for determining a subject on a bed to be in an abnormality state, wherein
machine learning is performed based on a frequency feature amount calculated by performing short-time Fourier transform on a load of the subject detected by a load sensor disposed at the bed, and
a computer is caused to function so as to determine the subject to be in the abnormality state based on the frequency feature amount.

23. The trained model according to claim 22, wherein the frequency feature amount is a mean amplitude value in a predetermined frequency band of a frequency spectrum.

24. The trained model according to claim 23, wherein the mean amplitude value in the predetermined frequency band includes a mean amplitude value in a first frequency band and a mean amplitude value in a second frequency band at a higher frequency side than the first frequency band.

25. The trained model according to claim 24, wherein the first frequency band is included in a band of 0.1 to 1.0 Hz, and the second frequency band is included in a band of 3.6 to 5.5 Hz.

26. The trained model according to claim 24 or 25, wherein the mean amplitude value in the predetermined frequency band further includes a mean amplitude value in a third frequency band at a higher frequency side than the first frequency band and at a lower frequency side than the second frequency band.

27. The trained model of any one of claims 22 to 26, wherein the abnormality state includes a coughing state and/or an apneic state.

28. A non-transitory computer-readable recording medium for storing the trained model according to any one of claims 22 to 27.

29. An abnormality determination model generation method for determining a subject on a bed to be in an abnormality state, the abnormality determination model generation method comprising:
detecting a load of the subject by a load sensor disposed at the bed;
calculating a frequency feature amount by performing short-time Fourier transform on the load of the subject; and
generating an abnormality determination model for determining the subject to be in the abnormality state based on the frequency feature amount, through unsupervised machine learning using the frequency feature amount, wherein
the abnormality state includes a coughing state and an apneic state, and
the frequency feature amount includes a mean amplitude value of a frequency spectrum in a first frequency band included in a band of 0.1 to 1.0 Hz and a mean amplitude value of a frequency spectrum in a second frequency band included in a band of 3.6 to 5.5 Hz.
